**Europäisches Patentamt**

(19) **European Patent Office**

**Office européen des brevets**

(11) Veröffentlichungsnummer: **0 028 397**
**B1**

(12) # EUROPÄISCHE PATENTSCHRIFT

(45) Veröffentlichungstag der Patentschrift:
**12.09.84**

(51) Int. Cl.³: **G 01 R 33/06, A 61 C 19/04**

(21) Anmeldenummer: **80106651.5**

(22) Anmeldetag: **29.10.80**

(54) **Verfahren zur Beseitigung des Einflusses von Remanenz in Empfangssystemen und Vorrichtung zur Durchführung des Verfahrens.**

(30) Priorität: **03.11.79 DE 2944490**

(43) Veröffentlichungstag der Anmeldung:
**13.05.81 Patentblatt 81/19**

(45) Bekanntmachung des Hinweises auf die Patenterteilung:
**12.09.84 Patentblatt 84/37**

(84) Benannte Vertragsstaaten:
**FR GB IT**

(56) Entgegenhaltungen:
**AT - B - 209 423**
**DE - A - 2 715 106**
**DE - A - 2 814 551**

(73) Patentinhaber: **SIEMENS AKTIENGESELLSCHAFT,**
**Berlin und München Wittelsbacherplatz 2,**
**D-8000 München 2 (DE)**

(72) Erfinder: **Schorr, Wolfgang, Walter-Rathenaustrasse 26,**
**D-6148 Heppenheim (DE)**

## Beschreibung

Die Erfindung bezieht sich auf ein Verfahren zur Beseitigung des Einflusses von Remanenz in Empfangssystemen, bei denen zur Erfassung eines von einem Magnetfelderzeuger ausgehenden Magnetflusses bzw. einer Magnetflussänderung ein oder mehrere Magnetflussaufnehmer aus ferromagnetischem Werkstoff, vorzugsweise mit Antennen versehene Hallgeneratoren, vorgesehen sind. Weiterhin bezieht sich die Erfindung auf eine Verwendung dieses Verfahrens sowie eine Vorrichtung zu seiner Durchführung.

In Empfangssystemen, bei denen insbesondere relativ kleine Magnetfeldänderungen mit Hilfe von Magnetflussaufnehmern, wie Hallgeneratoren, erfasst werden sollen und bei denen reproduzierbare Messergebnisse mit aus einer Feldflussänderung gewonnenen Signalen zu erzielen sind, dürfen keine störenden, sich auf die Auswertung der Signale nachteilig auswirkenden Remanenzerscheinungen auftreten. Solche störenden Remanenzerscheinungen treten jedoch wegen der in der Regel nicht absolut remanenzfreien ferromagnetischen Materialien der Magnetflussaufnehmer (Hallgeneratoren und gegebenenfalls mit diesen gekuppelte Antennen) bei Magnetfeldänderungen, z.B. bei einer Ortsveränderung des Empfangssystems im Erdmagnetfeld oder bei Annäherung eines Messinstruments mit einem weiteren Magnetfelderzeuger, auf.

Ein Anwendungsbeispiel wird in der Gnathologie gesehen, wo man mit Hilfe einer Messvorrichtung die Bewegung des Unterkiefers eines Menschen erfassen und aufzeichnen will. Dabei wird am Unterkiefer eines Patienten ein Magnetfelderzeuger, z.B. ein Permanentmagnet, befestigt und am Kopf des Patienten und in einem Abstand zum Magnetfelderzeuger ein Magnetflussaufnehmersystem mit Hallgeneratoren und Antennen angeordnet. Die bei einer Bewegung des Unterkiefers auftretenden Magnetfeldänderungen werden vom Aufnehmersystem in allen drei Bewegungsebenen erfasst und über eine elektronische Auswerteinrichtung ausgewertet. Der Aufbau einer solchen Einrichtung ist beispielsweise in der nach dem Prioritätstag der vorliegenden Anmeldung veröffentlichten deutschen Patentanmeldung gemäss DE-A-2 852 764 oder in DE-A-2 814 551 beschrieben.

Um insbesondere bei solchen Messvorrichtungen eindeutige und reproduzierbare Messergebnisse zu bekommen ist es notwendig, dass keine störenden, den Ausgangspunkt (Nullpunkt) des Messsystems verändernden Remanenzerscheinungen auftreten.

Nachdem es aber praktisch keine absolut remanenzfreien ferromagnetischen Materialien für die Magnetflussaufnehmer (Hallgeneratoren und Antennen) gibt und auch das Erdmagnetfeld sich störend auf den Messvorgang auswirkt, ist anzustreben, den Einfluss der Remanenz zu beseitigen.

Ein denkbarer Weg zur Beseitigung dieser störenden Remanenzerscheinungen wäre eine Entmagnetisierung der Flussaufnehmer. In der Veröffentlichung «Kuhrt/Lippmann, Hallgeneratoren», Springer-Verlag 1968, Seiten 32, 33, ist ein solcher Weg aufgezeigt. Das Verfahren hierzu besteht darin, dass man ein magnetisches Wechselfeld mit einer Amplitude $H > H_s$ auf den ferromagnetischen Werkstoff einwirken lässt und die Amplitude dieses Wechselfeldes langsam zurücknimmt. Die Magnetisierung umfährt so auf immer kleiner werdenden Hystereseschleifen den Koordinatenursprung und erreicht schliesslich bei verschwindender Wechselfeldamplitude den Punkt $H = 0$, $M = 0$.

Bei diesem Entmagnetisierungsverfahren, das relativ zeitaufwendig ist, darf kein äusseres Feld einwirken. Eine exakte Entmagnetisierung ist also nur im feldfreien Raum möglich. Um das Erdmagnefeld zu umgehen, ist eine Entmagnetisierung demnach nur senkrecht zum Erdmagnetfeld und ohne Messmagnet durchführbar. Für viele Anwendungsfälle ist dieses Verfahren also nicht geeignet.

Aufgabe der vorliegenden Erfindung ist es, ein demgegenüber verbessertes Verfahren zur Beseitigung des Einflusses von Remanenz sowie eine Vorrichtung zur Durchführung des Verfahrens anzugeben, das unabhängig vom vorhandenen Erdmagnetfeld und anderen, eventuell störenden Feldeinflüssen, wie z.B. eines Messmagneten od.dgl., arbeitet und mit dem eindeutige und reproduzierbare Messergebnisse zu erzielen sind.

Das gestellte Ziel wird gemäss der Erfindung mit dem Verfahren nach Anspruch 1 bzw. der Vorrichtung nach Anspruch 8 erreicht. Die Verwendung des Verfahrens ist in Anspruch 7 definiert.

Im Gegensatz zu einem anderen bekannten Verfahren (AT-B-209 423), welches zur Messung von unter anderem schwachen Gleichfeldern vorgesehen ist und bei welchem das Aufmagnetisieren der Kerne bis zur Sättigungsgrenze zum Messprinzip gehört, werden gemäss der Erfindung die Magnetflussaufnehmer vor einer Messwerterfassung mit Gleichmagnetfeldimpulsen beaufschlagt. Beim erfindungsgemässen Verfahren dient also das Aufmagnetisieren zur Vorbereitung einer Messung, um dann für die Messung jeweils gleiche Ausgangsposition zu haben.

Durch die Beaufschlagung des Magnetaufnehmersystems in den Impulsintervallen mit Magnetisierungsimpulsen, gebildet durch kurze Gleichstromimpulse bestimmter Impulsgrösse, wie vorgeschlagen, ist gewährleistet, dass die Ausgangsspannungen an den Magnetflussaufnehmern gleichen Anfangsbedingungen unterliegen. Nachdem der Zwischenimpuls nach jedem Messimpuls, mit dem die Magnetflussaufnehmer beaufschlagt werden, wiederholt wird, werden stets gleiche Anfangsbedingungen geschaffen. Nach Impulsende fällt die Magnetisierung auf den Wert $M_R$, der die maximal mögliche Remanenz des verwendeten Materials eines Magnetflussaufnehmers darstellt, zurück. Dieser Wert ist unabhängig von eventuell vorkommenden Fremdfeldern.

Vorteilhafte Weiterbildungen und Ausgestaltungen der Erfindung sind in den abhängigen Ansprüchen enthalten. Anhand der Figuren wird das Verfahren näher erläutert und eine Vorrichtung zur Durchführung des Verfahrens aufgezeigt. Es zeigen:

Fig. 1 eine Vorrichtung zur Erfassung der Bewegung des Unterkiefers eines Patienten,

Fig. 2 ein Prinzipschaltbild zur Ansteuerung einerseits eines Hallgenerators mit pulsförmigen Signalen und anderseits einer Magnetisierungsspule mit zeitlich dazu versetzten Impulsen,

Fig. 3 eine Impulsdiagramm nach dem erfindungsgemässen Verfahren,

Fig. 4 eine Hystereseschleife eines ferromagnetischen Werkstoffes.

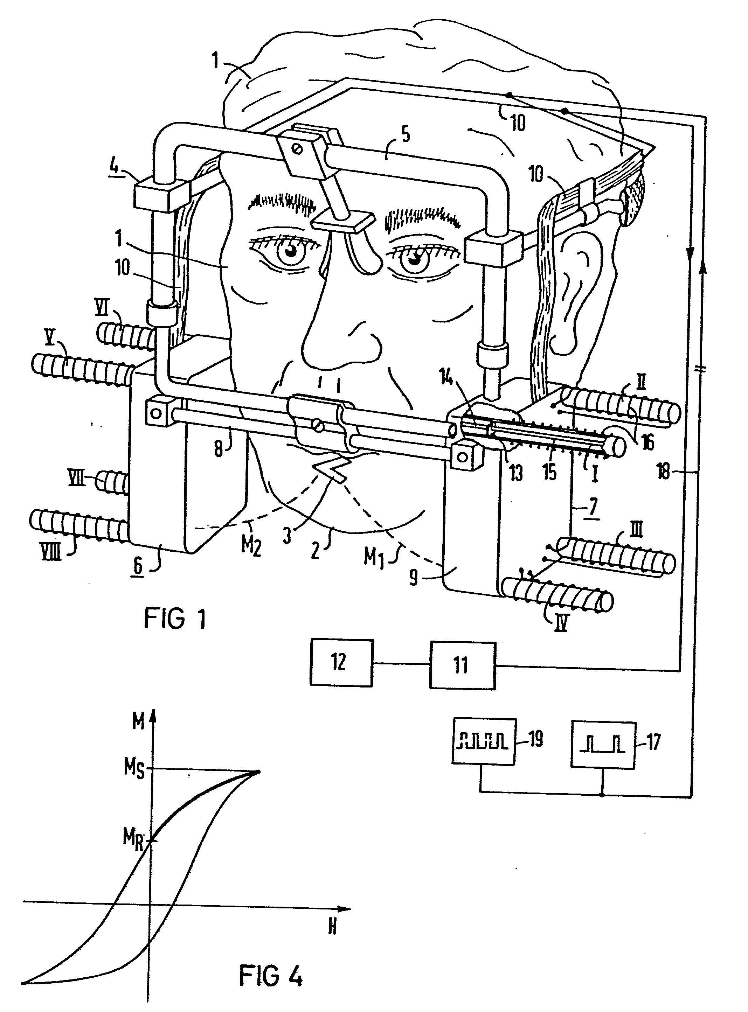

Die Fig. 1 zeigt in einer schaubildlichen Darstellung eine Vorrichtung zur Bestimmung des Ortes, der Lage und/oder einer orts- bzw. Lageänderung eines Punktes des Unterkiefers eines Patienten. In der Figur ist mit 1 der Kopf eines Patienten und mit 2 dessen Unterkiefer bezeichnet. Mit 3 ist ein als Felderzeuger dienender Permanentmagnet bezeichnet, der innerhalb der Mundhöhle des Patienten an einer beliebigen Stelle des Unterkiefers durch geeignete Haft- oder Klebemittel befestigt wird. Der Magnetfelderzeuger 3 besteht aus zwei gleichdimensionierten Stabmagneten, wie sie in der deutschen Patentanmeldung DE-A-2 715 106 näher beschrieben sind. Der Magnetfelderzeuger 3 erzeugt zwei in der Figur gestrichelt angedeutete, unregelmässige, nicht rotationssymmetrische Magnetfelder $M_1$, $M_2$.

Ausserhalb des Patientenmundes befindet sich eine Magnetflussaufnehmeranordnung 4, bestehend im wesentlichen aus einem am Patientenkopf 1 gehaltenen Gestell 5 und einem Aufnehmersystem mit einem links und rechts vom Unterkiefer befindlichen Aufnehmerblock 6 und 7. Das Gestell 5 ist in bekannter Weise als kombiniertes Brillen- oder Kopfgestell ausgebildet und enthält mehrere, nicht näher bezeichnete Gelenke zur Anpassung an die unterschiedlichen Kopfkonstellationen des Patienten. Die beiden Aufnehmerblöcke 6 und 7 sind durch eine mit dem Gestell 5 verbundene Stange 8 starr miteinander verbunden.

Jeder der Aufnehmerblöcke 6, 7 enthält vier Magnetflussaufnehmer I bis VIII, die in einem Kunststoffgehäuse 9 gehalten sind, und zwar so, dass sie jeweils parallel zueinander liegen. Die von den nachfolgend noch näher beschriebenen Magnetflussaufnehmern I bis VIII aufgenommenen Signale werden über mehradrige Leitungen 10 einer elektronischen Auswerteinrichtung 11 und von dort aus weiter einer geeigneten Indikatoreinrichtung 12 zugeführt.

Zur Erläuterung des Aufbaus der Magnetflussaufnehmer I bis VIII ist der Magnetflussaufnehmer I teilweise im Schnitt dargestellt. Der Magnetflussaufnehmer enthält einen als Sensor dienenden, plättchenförmigen Hallgenerator 13, an dessen wirksame Fläche beidseitig verschieden lange Antennenstäbe 14 und 15 aus Mu-Metall anliegen. Bei einer Bewegung des Unterkiefers ändert sich der von den Hallgeneratoren 13 erfasste Feldfluss. Die von den Hallgeneratoren dadurch erzeugten Signale werden über einen im Gehäuse 9 angeordneten, in der Figur nicht dargestellten Vorverstärker der Elektronikeinheit 11 zugeführt, wo die Signale dann für eine bildliche Darstellung aufbereitet werden.

Um jeden Magnetflussaufnehmer ist einlagig eine Induktionsspule 16 gewickelt. Die einlagige Umwicklung der Flussaufnehmer hat den Vorteil, dass einerseits eine gute Wärmeabfuhr gegeben ist und anderseits ein besseres Magnetfeld zur Aufmagnetisierung erzeugt wird. Die vier Spulen 16 eines jeden Blockes (6 bzw. 7) sind hintereinandergeschaltet und mit den vier anderen des anderen Blockes parallel geschaltet. Wie später noch näher erläutert, werden die Spulen 16 von einem Impulsgenerator 19 über den einen Leitungsstrang einer Doppelleitung 18 mit periodischen Rechteckimpulsen versorgt. Mit 17 ist ein weiterer Impulsgenerator bezeichnet, der über den anderen Leitungsstrang der Doppelleitung 18 die Hallgeneratoren 13 mit pulsförmigen Signalen ansteuert.

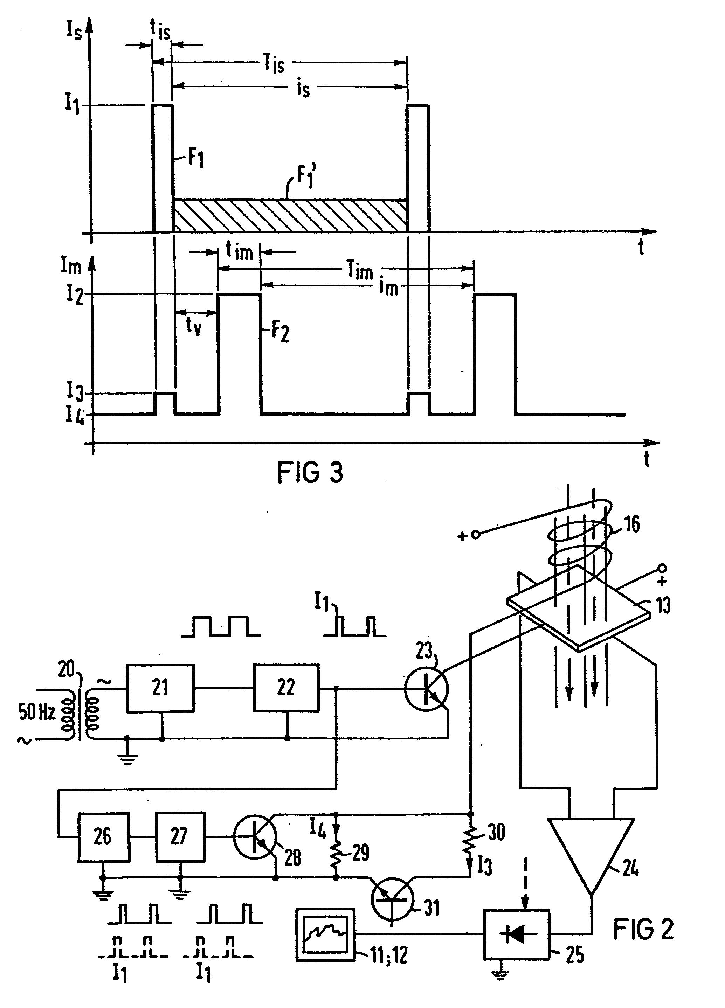

Die Fig. 2 zeigt anhand eines Blockschaltbildes eine insbesondere für sehr kleine Feldstärken vorteilhafte Ansteuerschaltung für die Hallgeneratoren 13 sowie ein Prinzipschaltbild zur Ansteuerung der Spulen 16 mit Impulsen.

Von einem Wechselstromtransformator 20 werden Wechselstromsignale mit der Frequenz von 50 Hz erzeugt, die zur Impulsformung zunächst einem Schmitt-Trigger 21 zugeführt werden. Eine monostabile Kippstufe 22 liefert dann Ansteuerimpulse $I_1$ in einem bestimmten Tastverhältnis, die anschliessend über einen Transistor 23 dem Hallgenerator 13 zugeführt werden.

Die durch die aufgezeigte Schaltung erzielbaren Impulssignale geben nun eine Information über die Grösse bzw. Form (Hüllkurve) des Nutzsignals, welches aus den Hallgeneratoren 13 erhalten wird. Um die Impulse in ein Nutzsignal umzuwandeln, werden diese zunächst über einen Verstärker 24 einem Spitzenwertgleichrichter 25 zugeführt. Die hieraus erhältlichen Signale können sodann mit Hilfe einer geeigneten Auswerte-(Rechner) und Indikatoreinrichtung 11, 12 ausgewertet und optisch dargestellt werden.

In dem Impulsdiagramm nach Fig. 3 sind die vom Taktgenerator 17 und beispielsweise mit Hilfe der Schaltungsanordnung nach Fig. 2 erzeugten Impulse mit $I_1$ bezeichnet. Im Anwendungsfall beträgt die Impulsbreite $t_{is}$ etwa 0,5 ms bei einer Impulsperiode $T_{is}$ von 20 ms. Das Verhältnis Impulsbreite zu Impulsintervall $t_{is}/i_s$ ist im vorliegenden Falle so gewählt, dass die erlaubte effektive Steuerleistung des Hallgenerators nicht überschritten wird, d.h. die mit $F_1$ bezeichnete Fläche darf nicht grösser sein als die mit $F_{1'}$ bezeichnete Fläche, die der Steuerleistung des Hallgenerators bei Gleichstrom entspricht. Je extremer das Tastverhältnis, d.h. je kürzer die Impulse und je länger die Impulspause gewählt sind, um so höher kann die maximale Amplitude des Steuerstromes angesetzt und damit die Empfindlichkeit des Hallgenerators gesteigert werden.

Im Impulsintervall $i_s$, also in den Impulspausen, werden den Spulen 16 mit Hilfe des Impulsgenerators 19 periodisch kurze Gleichstromimpulse $I_2$ zugeführt. In Fig. 2 ist ein Schaltbild zur Erzeugung dieser Impulse aufgezeigt. Die Ansteuerimpulse $I_1$ für die Hallgeneratoren 13 werden einer monostabilen Kippstufe 26 zugeführt, mit deren abfallender Impulsflanke eine weitere monostabile Kippstufe 27 angesteuert wird. Die Kippstufe 26 gibt die in dem Impulsdiagramm nach Fig. 3 mit $t_V$ bezeichnete Verzögerungszeit gegenüber dem Steuerimpuls $I_1$ an, die Kippstufe 27 die Impulsbreite, mit der der im Schaltbild nach Fig. 2 mit 28 bezeichnete Transistor und damit die Magnetisierungsspulen 16 angesteuert werden. Die Impulse $I_2$ werden synchron zu den Steuerimpulsen $I_1$, im vorliegenden Fall mit einer Frequenz von 50 Hz, zugeführt. Der Zeitpunkt, in welchem die Impulse $I_2$ zugeführt werden, kann an und für sich beliebig gewählt werden, er muss nur innerhalb der Impulspause $i_s$ liegen. Impulshöhe und Impulsbreite sind dabei so gross gewählt, dass mit den durch die Spulen 16 induzierten Magnetisierungsimpulsen die Sättigungsfeldstärke für die Flussaufnehmer (Hallgenerator und Antennen) erreicht wird. Die Magnetisierung des Antennensystems erreicht dadurch ihren Sättigungswert $M_s$, entsprechend der Darstellung in Fig. 4, die eine Hysteresekurve eines ferromagnetischen Werkstoffes zeigt. Durch den periodisch in dem Impulsintervall $i_s$ zugeführten Gleichstromimpuls $I_2$ lässt sich also stets die Sättigungsfeldstärke des für die Aufnehmer vorgesehenen Werkstoffes erreichen. Nach Impulsende fällt die Magnetisierung auf den Wert $M_r$ zurück. Dieser Wert ist, da er die maximal mögliche Remanenz für das System darstellt, unabhängig von eventuell vorkommenden Fremdfeldern. Nachdem eine Wiederholung des Vorganges nach jedem Messimpuls, im vorliegenden Falle also alle 20 ms, erfolgt, ist gewährleistet, dass die Ausgangsspannung an den Hallgeneratoren 13 immer gleichen Anfangsbedingungen unterliegt.

Das erfindungsgemässe Verfahren ist auch bei nicht gepulstem Steuerstrom anwendbar. Bei gleichförmigem Steuerstrom werden dann die Magnetisierungsimpulse an beliebiger Stelle in periodischer Folge zugeführt, wobei der Steuerstrom oder die Messspannung dann während der Dauer der Magnetisierungsimpulse ausgetastet wird.

Die Spulen 16 auf jedem Flussaufnehmer I bis VIII sind geometrisch gleich, d.h. sie haben gleiche Windungszahlen, Durchmesser und Längen. Schaltet man während der Messzeit $t_{is}$ auf allen Aufnehmern einen weiteren, kurzen Gleichstromimpuls $I_3$ von definierter Grösse auf, wie dies in dem Impulsdiagramm nach Fig. 3 aufgezeigt ist, so werden jeweils gleich Magnetfelder an den Hallgeneratoren 16 erzeugt. Mit Hilfe von dafür vorgesehenen Anpassverstärkern können so die Hallgeneratoren auf gleiche Empfindlichkeit abgeglichen werden. Durch diese Massnahme lässt sich ein vereinfachter Abgleich und auch eine leichtere Kontrolle des Abgleichs erzielen. Die Gleichstromimpulse $I_3$ entstehen durch Parallelschalten eines Widerstandes 30 über einen Transistor 31 zum Transistor 28. Während einer Messung ist der Gleichstrom $I_3$ nicht vorhanden.

Um den sog. «Umkehrfehler» bei einer Feldumkehr zu vermeiden (vgl. Veröffentlichung Kuhrt/Lippmann, Seiten 143/144) wird zum Abgleich der Einrichtung bzw. zur Überprüfung des Abgleiches in einer vorteilhaften Ausgestaltung der Erfindung vorgeschlagen, die Spulen 16 durch Parallelschalten eines Widerstandes 29 zum Transistor 28 mit Gleichstrom von gleicher Grösse und Polarität ($I_4$ in Fig. 3) zu beaufschlagen. An den Verstärkerausgängen wird so – vorausgesetzt, dass diese im feldfreien Zustand auf «Null» abgeglichen wurden – eine sog. Offset-Spannung erzeugt. Wählt man die Stromstärke, die der Felstärke einer jeden Einzelspule entspricht, so hoch, dass sie grösser ist als die Summe der Feldstärke von Erdmagnetfeld und Magnetfeld, so wird es am Hallgenerator zu keiner Zeit zu einer Feldumkehr und damit zu einem Umkehrfehler kommen.

## Patentansprüche

1. Verfahren zur Beseitigung des Einflusses von Remanenz in Empfangssystemen, bei denen zur Erfassung eines von einem Magnetfelderzeuger (3) ausgehenden Magnetflusses (M1, M2) bzw. einer Magnetflussänderung ein oder mehrere Magnetflussaufnehmer (I–VIII) aus ferromagnetischem Werkstoff, vorzugsweise mit Antennen (14, 15) versehene Hallgeneratoren (13), vorgesehen sind, dadurch gekennzeichnet, dass die Magnetflussaufnehmer (I–VIII) jeweils vor einer Messwerterfassung in konstanten zeitlichen Abständen mit Gleichmagnetfeldimpulsen ($I_2$) beaufschlagt werden, deren Impulshöhe und -breite ($t_{im}$) so gewählt sind, dass damit die Sättigungsfeldstärke ($M_s$) der Magnetflussaufnehmer erreicht wird.

2. Verfahren nach Anspruch 1, dadurch gekennzeichnet, dass die Magnetflussaufnehmer (I–VIII) aus Hallgeneratoren (13) bestehen, die mit Messstromimpulsen ($I_1$) beaufschlagt werden, und dass die Gleichmagnetfeldimpulse ($I_2$) jeweils in den Impulsintervallen ($i_s$) dieser Messstromimpulse in gleichen Abständen zu den Messstromimpulsen ($I_1$) zugeführt werden.

3. Verfahren nach Anspruch 1 oder 2, dadurch gekennzeichnet, dass die Gleichmagnetfeldimpulse ($I_2$) mit der Netzfrequenz von 50 bzw. 60 Hz zugeführt werden.

4. Verfahren nach einem der Ansprüche 1 bis 3, dadurch gekennzeichnet, dass die Gleichmagnetfeldimpulse ($I_2$) Rechteckimpulse sind.

5. Verfahren nach einem der Ansprüche 2 bis 4, dadurch gekennzeichnet, dass bei Verwendung mehrerer Magnetflussaufnehmer (I–VIII) während der Zeitdauer ($t_{is}$) der Messstromimpuls ($I_1$) jeder Magnetflussaufnehmer einem weiteren Gleichmagnetfeldimpuls ($I_3$) jeweils gleicher Grösse ausgesetzt wird.

6. Verfahren nach Anspruch 2, dadurch gekennzeichnet, dass an die Hallgeneratoren (13) neben den Gleichmagnetfeldimpulsen ($I_2$) ein in Grösse und Polarität für alle Hallgeneratoren gleicher Gleichstrom ($I_4$) angelegt wird.

7. Verwendung des Verfahrens nach einem der Ansprüche 1 bis 6 in einer Vorrichtung zur Erfassung der Bewegung des Unterkiefers eines Patienten, wobei die Vorrichtung ein in fester Relation zu einem Patientenkopf (1) angeordnetes Gestell (5) mit mehreren Magnetflussaufnehmern (I–VIII) enthält, die den Magnetfluss bzw. eine Magnetflussänderung von einem im Abstand von den Magnetflussaufnehmern am Unterkiefer (2) des Patienten angeordneten Magnetfelderzeuger (3) erfassen.

8. Vorrichtung zur Durchführung des Verfahrens nach einem der Ansprüche 1 bis 6, mit einem in fester Relation zu einem Patientenkopf (1) angeordneten Gestell (5), an dem in Montageblöcken (6, 7) zusammengefasst mehrere Magnetflussaufnehmer (I–VIII) angeordnet sind, dadurch gekennzeichnet, dass an dem Gestell (5) symmetrisch bezüglich des Patientenkopfs (1) zwei Magnetflussaufnehmer-Montageblöcke (6, 7) mit jeweils vier Magnetflussaufnehmern befestigt sind, dass jeder Magnetflussaufnehmer (I–VIII) jeweils von einer Induktionsspule (16) umgeben ist, die jeweils von einem Generator (19) mit Gleichstromimpulsen gespeist ist und Gleichmagnetfeldimpulse liefert, deren Impulshöhe und -breite ($t_{im}$) so gewählt sind, dass damit die Sättigungsfeldstärke ($M_s$) der Magnetflussaufnehmer erreicht wird.

9. Vorrichtung nach Anspruch 8, dadurch gekennzeichnet, dass die Induktionsspulen (16) einlagig um die Magnetflussaufnehmer (I–VIII) angeordnet sind.

10. Vorrichtung nach Anspruch 9, dadurch gekennzeichnet, dass die Induktionsspulen (16) geometrisch gleich ausgebildet sind.

## Claims

1. A method of eliminating the influence of remanence in receiving systems in which, in order to detect a magnetic flux (M1, M2) emanating from a magnetic flux generator (3) or in order to detect a change in magnetic flux there are provided one or more than one magnetic flux receivers (I–VIII) composed of ferromagnetic material, preferably Hall generators (13), provided with antennae (14, 15), characterised in that prior to the detection of a measured value the magnetic flux receivers (I–VIII) are each fed at constant time intervals with constant magnetic field pulses ($I_2$) whose pulse level and width ($t_{im}$) are such that the magnetic flux receivers attain their saturation field strength ($M_s$).

2. A method as claimed in Claim 1, characterised in that the magnetic flux receivers (I–VIII) consist of Hall generators (13) which are supplied with measuring current pulses ($I_1$), and that each of the constant magnetic field pulses ($I_2$) is supplied in the pulse interval ($i_s$) between adjacent measuring current pulses, at equal intervals to the measuring current pulses ($I_1$).

3. A method as claimed in Claim 1 or Claim 2, characterised in that the constant magnetic field pulses ($I_2$) are supplied at the mains frequency of 50 or 60 Hz, as the case may be.

4. A method as claimed in one of Claims 1 to 3, characterised in that the constant magnetic field pulses ($I_2$) are rectangular pulses.

5. A method as claimed in one of Claims 2 to 4, characterised in that when a plurality of magnetic flux receivers (I–VIII) is used, for the duration ($t_{is}$) of the measuring current pulses ($I_1$) each magnetic flux receiver is exposed to a further constant magnetic field pulse ($I_3$) of equal magnitude.

6. A method as claimed in Claim 2, characterised in that the Hall generators (13) are subjected to a direct current ($I_4$) equal in magnitude an polarity to the constant magnetic field pulses ($I_2$) for all the Hall generators.

7. In a device for detecting movement of the lower jaw of a patient, the use of the process as claimed in one of Claims 1 to 6 where the device contains a frame (5) which is arranged in a fixed relationship to a patient's head (1) and which is equipped with a plurality of magnetic flux receivers (I–VIII) which detect the magnetic flux or a change in magnetic flux of a magnetic field generator (3) which is attached to the lower jaw (2) of the patient at an interval from the magnetic flux receivers.

8. A device for implementing the process claimed in one of Claims 1 to 6, with a frame (5) which is arranged in a fixed relationship to a patient's head (1) and which is equipped with a plurality of magnetic flux receivers (I–VIII) combined in assembly blocks (6, 7), characterised in that two magnetic flux receivers assembly blocks (6, 7) each consisting of four magnetic flux receivers are attached to the frame (5) symmetrically in relation to the patient's head (1), that each magnetic flux receiver (I–VIII) is surrounded by an induction coil (16) which is in each case fed with d.c. pulses by a generator (19) and supplies constant magnetic field pulses whose pulse level and width ($t_{im}$) are such that the magnetic flux receivers attain their saturation field strength ($M_s$).

9. A device as claimed in Claim 8, characterised in that the induction coils (16) are arranged in one layer around the magnetic flux receivers (I–VIII).

10. A device as claimed in Claim 9, characterised in that the induction coils (16) are designed to be geometrically identical.

## Revendications

1. Procédé pour supprimer l'influence de la rémanence dans des systèmes de réception, dans lesquels, pour la saisie d'un flux magnétique (M1, M2) émanant d'un générateur de flux magnétique (3), ou d'une variation du flux magnétique, on prévoit un ou plusieurs récepteurs de flux magnétique (I–VIII), en un matériau ferromagnétique, de préférence des générateurs de Hall (13) pourvus d'antennes (14, 15), caractérisé par le fait que les récepteurs de flux magnétique (I–VIII) sont respectivement alimentés, avant une saisie de valeur de mesure, et à des distances temporelles constantes, avec des impulsions d'un aimant magnétique continu ($I_2$), dont la hauteur et la largeur d'impulsions ($t_{im}$) sont choisies de telle façon qu'elles permettent d'atteindre l'intensité du champ de saturation ($M_s$) des récepteurs du flux magnétique.

2. Procédé selon la revendication 1, caractérisé par le fait que les récepteurs du flux magnétique (I–VIII) sont constitués par des générateurs de Hall (13) qui sont alimentés avec des impulsions d'un courant de mesure ($I_1$), et que les impulsions du champ magnétique continu ($I_2$) sont respectivement amenées, dans les intervalles d'impulsions ($i_s$) de ces impulsions du courant de mesure, et à des intervalles identiques, aux impulsions du courant de mesure ($I_1$).

3. Procédé selon la revendication 1 ou 2, caractérisé par le fait que les impulsions du champ magnétique continu ($I_2$) sont appliquées avec la fréquence du réseau de 50 ou de 60 Hz.

4. Procédé selon l'une des revendications 1 à 3, caractérisé par le fait que les impulsions du champ magnétique continu ($I_2Z$) sont des impulsions rectangulaires.

5. Procédé selon l'une des revendications 2 à 4, caractérisé par le fait que dans le cas de l'utilisation de plusieurs récepteurs du flux magnétique (I–VIII) et pendant la durée ($t_{is}$) des impulsions du courant de mesure ($I_1$), chaque récepteur du flux magnétique est exposé à une impulsion de champ magnétique continu supplémentaire ($I_3$) de même grandeur.

6. Procédé selon la revendication 6, caractérisé par le fait que l'on applique aux générateurs de Hall (13), en plus des impulsions du champ magnétique continu ($I_2$), un courant continu ($I_4$) qui est identique pour tous les générateurs de Hall du point de vue de la grandeur et de la polarité.

7. Mise en œuvre du procédé selon l'une des revendications 1 à 6 dans un dispositif pour saisir le mouvement de la mâchoire inférieure d'un patient, le dispositif comportant un bâti (5) à plusieurs récepteurs du flux magnétique (I–VIII), disposé suivant une relation fixe par rapport à la tête d'un patient (1), lesquels récepteurs du flux magnétique saisissent le flux magnétique ou une variation du flux magnétique d'un générateur de champ magnétique disposé sur la mâchoire inférieure (2) du patient, à une certaine distance des récepteurs du flux magnétique.

8. Dispositif pour la mise en œuvre du procédé selon l'une des revendications 1 à 6, avec un bâti (5), disposé suivant une relation fixe par rapport à la tête d'un patient (1), bâti sur lequel sont disposés plusieurs récepteurs de flux magnétique (I–VIII) assemblés en des blocs de montage (6, 7), caractérisé par le fait qu'au bâti sont fixés, symétriquement par rapport à la tête du patient (1), deux blocs de montage de récepteurs de flux magnétique (6, 7), avec respectivement quatre récepteurs de flux magnétique, que chaque récepteur de flux magnétique (I–VIII) est entouré par une bobine d'induction (16) qui est alimentée par un générateur (19) avec des impulsions de courant continu et fournit les impulsions du champ magnétique continu dont la hauteur et la largeur des impulsions ($t_{im}$) sont choisies de telle façon qu'elles permettent que soit atteinte l'intensité du champ de saturation ($M_s$) des récepteurs du flux magnétique.

9. Dispositif selon la revendication 8, caractérisé par le fait que les bobines d'induction (16) sont disposées, suivant une couche, autour des récepteurs du flux magnétique (I–VIII).

10. Dispositif selon la revendication 9, caractérisé par le fait que les bobines d'induction (16) sont réalisées avec une géométrie identique.

FIG 1

FIG 4

FIG 3

FIG 2